# EUROPEAN PATENT APPLICATION

(11) **EP 4 586 269 A1**
(43) Date of publication of application: **16.07.2025**
(21) Application number: 24150931.4
(22) Date of filing: 09.01.2024
(51) Int. Cl.: G16H 40/63, B60W 40/08, G06N 20/00, G16H 50/20, G16H 50/30, G16H 50/70

(54) **DRIVER ASSISTANCE SYSTEM**

(71) Applicant: Harman Becker Automotive Systems GmbH, 76307 Karlsbad (DE)
(72) Inventor: AMTHOR, Peter, 76307 Karlsbad (DE)
(74) Representative: Westphal, Mussgnug & Partner, Patentanwälte mbB

(57) **Abstract**

A method comprises, at the beginning of each driving session of a vehicle, determining one or more parameters related to a driver of the vehicle, storing the one or more parameters, thereby creating a temporary personal profile for the driver, and using artificial intelligence, AI, and based on the temporary personal profile, determining whether the driver is under the influence of impairing substances.

## Description

### TECHNICAL FIELD

The disclosure relates to a driver assistance system and related method.

### BACKGROUND

Driver assistance can include any support that is provided to a driver of a vehicle with the aim of increasing personal safety and enhancing driver experience. Driver assistance systems can be used in order to prevent accidents, for example. When a driver of a vehicle is under the influence of an impairing substance such as, e.g., alcohol, drugs, medication, etc., the driver's ability to drive may be severely affected, which significantly increases the risk of (fatal) accidents.

There is a need for a driver assistance system and related method that can efficiently determine a driver's ability to drive.

### SUMMARY

A method includes at the beginning of each driving session of a vehicle, determining one or more parameters related to a driver of the vehicle, storing the one or more parameters, thereby creating a temporary personal profile for the driver, and using artificial intelligence, AI, and based on the temporary personal profile, determining whether the driver is under the influence of impairing substances.

A driver assistance system includes a processor, the processor being configured to, at the beginning of each driving session of a vehicle, determine one or more parameters related to a driver of the vehicle, store the one or more parameters, thereby creating a temporary personal profile for the driver, and using artificial intelligence, AI, and based on the temporary personal profile, determine whether the driver is under the influence of impairing substances.

Other systems, features and advantages of the disclosure will be or will become apparent to one with skill in the art upon examination of the following detailed description and figures. It is intended that all such additional systems, methods, features and advantages included within this description be within the scope of the invention and be protected by the following claims.

### BRIEF DESCRIPTION OF THE DRAWINGS

The arrangements and methods may be better understood with reference to the following description and drawings. The components in the figures are not necessarily to scale, emphasis instead being placed upon illustrating the principles of the invention. Moreover, in the figures, the same reference numerals designate the same components throughout the different views.
Figure 1 schematically illustrates a driver assistance system according to embodiments of the disclosure in a vehicle.
Figure 2 schematically illustrates a driver assistance system according to embodiments of the disclosure.
Figure 3 schematically illustrates, in a flow diagram, a method according to embodiments of the disclosure.

### DETAILED DESCRIPTION

The driver assistance system and related method according to the various embodiments described herein can efficiently determine a driver's ability to drive.

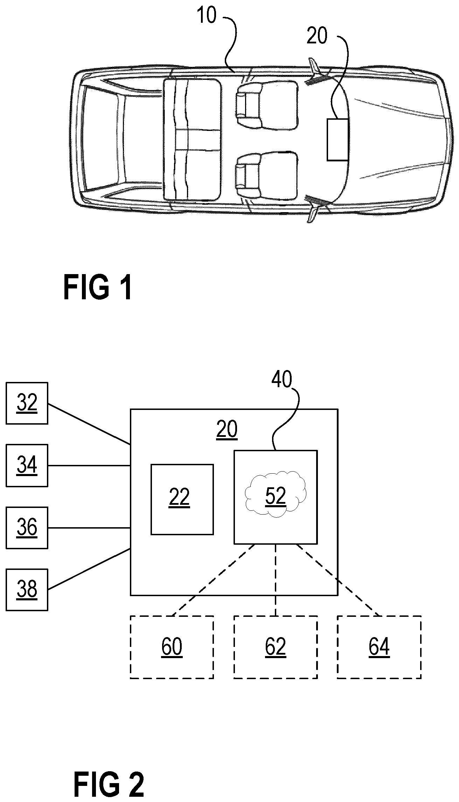

Figure 1 schematically illustrates a vehicle 10 comprising a driver assistance system 20. The driver assistance system 20 is schematically illustrated in further detail in Figure 2. The driver assistance system 20 comprises a processor 22, wherein the processor 22 is configured to, at the beginning of each driving session of a vehicle 10, determine one or more parameters related to a driver of the vehicle 10, store the one or more parameters, thereby creating a temporary personal profile 52 for the driver, and, using artificial intelligence, AI, and based on the temporary personal profile 52, determine whether the driver is under the influence of impairing substances.

The one or more parameters related to the driver may include at least one physiological parameter of the driver, for example. The at least one physiological parameter may comprise at least one of a heart rate, a respiratory rate, an intensity of transpiration, and a body temperature of the driver. When a driver is under the influence of impairing substances, such physiological parameters, as well as other physiological parameters, may be different as compared to a state in which the driver is not under the influence of impairing substances. For example, a heart rate and/or an intensity of transpiration may significantly change when a driver (or in general any person) is under the influence of impairing substances. Other parameters such as, e.g., physical parameters of the driver, may be taken into consideration as well when determining whether or not a driver is fit to drive. For example, a large and heavy person may perspire much more even under normal circumstances as compared to a small and lightweight person. That is, physical parameters of a driver such as, for example, a size, a weight, an age, and a gender of the driver, may be taken into consideration.

It is additionally also possible that determining one or more parameters related to a driver of the vehicle 10 further comprises determining one or more vehicle parameters related to a current driving behavior, and storing the one or more vehicle parameters in the temporary personal profile 52 of the driver. That is, it may be determined if the steering wheel is steady, or if the vehicle 10 is driven in a wiggly way, which may be an indication that the driver is under the influence of alcohol, for example. Additionally or alternatively it may be determined whether the vehicle 10 is driven at a comparably constant speed, or if the driver is not able to maintain a constant speed. This could also be an indication that the driver is under the influence of impairing substances. It is generally also possible to capture speech of a driver by means of one or more microphones arranged in the vehicle 10. The captured speech may be evaluated, as it may also be an indication whether a driver is under the influence of impairing substances (e.g., the driver may speak with a slur).

As is illustrated in Figure 2, the driver assistance system 20 may comprise or may be coupled to one or more sensors 32, 34, 36, 38 arranged in the vehicle 10, and determining the one or more parameters related to the driver of the vehicle 10 may comprise receiving one or more parameters from the one or more sensors 32, 34, 36, 38. The one or more sensors 32, 34, 36, 38 may comprise at least one of a camera 32, a radar sensor 34, a thermal imaging camera 36, and a weight sensor 38, for example. Additionally or alternatively, any other kind of sensor may be used with which physiological and/or physical parameters of a passenger may be determined.

By means of sensors 32, 34, 36, 38 arranged in the vehicle 10, physiological and/or physical parameters can be collected in a very safe and unobtrusive way. The driver may not even notice that the parameters are determined. For example, a size and weight of a driver may be determined or at least estimated by means of one or more cameras 32 (e.g., using suitable image processing techniques). One or more cameras are often already present in a vehicle for other applications, e.g., drowsiness detection applications. The weight of a driver may alternatively or additionally be determined by means of a weight sensor integrated in the respective vehicle seat, the driver is seated on. Such sensors are often already present in a vehicle for applications which detect the presence of passengers, in order to output a warning if a passenger does not wear their seatbelt. An age and a gender of a driver may be determined or at least estimated by means of cameras 32 and suitable image processing techniques, for example.

Physiological parameters such as, e.g., heart rate, respiratory rate, intensity of transpiration, and body temperature can be determined by means of cameras 32, radar sensors 34, and thermal imaging cameras 36, for example, without requiring any specific action of a driver. A heart rate and respiratory rate may be determined in a very accurate way by means of radar sensors 34, for example, while a thermal imaging camera 36 may be used to determine a body temperature. Any other suitable sensors, however, may alternatively or additionally used to determine physiological parameters of a driver.

Many people today have at least one permanent personal profile stored in a cloud or social network 64, for example. Many people today also use wearable devices such as fitness trackers 62 (e.g., smart watches or dedicated fitness tracking wristbands) which require a permanent personal profile to be stored either in the device itself or in a corresponding cloud. Such permanent personal profiles may include physical parameters such as, e.g., age, gender, size, weight, etc. The driver assistance system 20 may be coupled to a fitness tracking device 62 of a driver, or to a cloud or social network 64 and receive one or more physical parameters from the fitness tracking device 62, cloud or social network 64. Such parameters may be permanently stored in a permanent profile, as they most likely do not change significantly over time, or, if parameters such as, e.g., weight, change, they are usually updated by the user accordingly. A fitness tracking device 62 or other wearable sensors 60 such as, e.g., wearable electrocardiogram, ECG, devices, may also provide physiological parameters such as, e.g., heart rate, respiratory rate, etc., to the driver assistance system 20. The driver assistance system 20 may store parameters received from the one or more sensors 32, 34, 36, 38 arranged in the vehicle 10, and parameters received from other external devices (e.g., wearable sensor(s) 60, fitness tracking device(s) 62, cloud or social network 64) in the respective temporary personal profile 52. Accessing any external databases may require identification of the driver (e.g., by means of face recognition techniques). Further, a driver may be required to enter a password of the external database, or a password may be known to the driver assistance system 20 such that it can access the external database, when necessary.

The parameters related to the driver of the vehicle 10 are determined at least once at the beginning of a driving session. For example, the parameters may be determined and a temporary personal profile 52 may be stored when an engine of the vehicle 10 is started. The driver assistance system 20 may further comprise or may be coupled to a storage device 40, wherein the temporary personal profile 52 of the driver of the vehicle 10 is stored in the storage device 40. Determining the parameters and creating a temporary personal profile 52 when the engine of the vehicle 10 is started, however, is only one example. According to another example, the parameters may be determined and a temporary personal profile 52 may be stored when the engine of the vehicle 10 has been started and the vehicle 10 begins to drive. It is, however, generally also possible that the parameters are determined and the temporary personal profile 52 is stored when it is determined that a person is seated on a driver's seat of the vehicle 10, before the vehicle 10 is even started and/or begins to move.

When, at the beginning of a driving session it is determined that the driver may be under the influence of impairing substances and may not be fit to drive, an action may be taken. This may include informing the driver of their inability to drive and/or preventing the vehicle 10 from being driven by the concerned driver. It is, however, also possible that at the beginning of driving session a driver is considered fit to drive the vehicle 10. The driver, however, may consume substances (e.g., alcohol, drugs, medication) during the driving session such that at some point during the driving session they are no longer considered fit to drive. Therefore, it is possible that the temporary personal profile 52 of the driver of the vehicle 10 is updated at least once during a driving session. According to one example, the temporary personal profile 52 may be updated in regular intervals, e.g., every 10 minutes, or every 30 minutes. Alternatively or additionally, it is, however, also possible that the temporary personal profile 52 is updated upon occurrence of a triggering event.

An action that is taken when it is detected that the driver may be under the influence of impairing substances may be determined based on different factors. Actions that may or should be taken may depend on specific laws applying in a country the vehicle 10 is driven in. The action may further depend on the vehicle itself, i.e. whether the vehicle 10 is equipped with certain applications. For example, a low-range vehicle may not be equipped with means to automatically stop and park the vehicle when it is detected during a driving session that the driver is no longer fit to drive. A high-range vehicle, however, may comprise such means. The presence of certain vehicle applications may also depend on the make of the vehicle, for example. Some car manufacturers may implement certain features, while others do not.

As has been mentioned above, it is determined whether the driver is under the influence of impairing substances using artificial intelligence, AI (i.e. generative AI). For example, determining whether the driver is under the influence of impairing substances (determining a driving ability of the driver) based on the temporary personal profile 52 may comprise evaluating the temporary personal profile 52 by means of the (generative) artificial intelligence, AI, thereby creating a current driver profile. According to some embodiments of the disclosure, determining whether the driver is under the influence of impairing substances based on the temporary personal profile 52 further comprises comparing the current driver profile to an expected driver profile, wherein the expected driver profile comprises parameters related to a driver of the vehicle 10 and/or parameters of the vehicle 10 and collected during previous driving sessions, and/or the expected driver profile comprises parameters that are generally expected for drivers that are not under the influence of impairing substances and are considered fit to drive.

That is, according to some examples, a current (physical) state of the driver and a current driving behavior is compared to a normal (physical) state of the driver and a normal driving behavior of this specific driver which have been monitored during previous driving sessions. The (generative) artificial intelligence may collect, evaluate and store data from every driving session, and may compare data of a current driving session to data of some or all previous driving sessions. If, during a driving session, it is detected that the driver may be under the influence of impairing substances and is not fit to drive, the data of such driving session may not be stored, as it does not represent a normal (physical) state of the driver, for example. Alternatively or additionally, a current driver profile may be compared to a standardized driver profile which can be determined based on parameters (e.g., physiological and/or physical parameters of a driver, typical driving behavior, etc.) that are generally expected for a driver that is not under the influence of impairing substances.

(Generative) artificial intelligence is generally capable of evaluating parameters related to a driver of a vehicle 10 using (generative) models. (Generative) AI models learn the patterns and structure of their input training data and may then compare any new data to the input training data and determine whether or not the new data has similar characteristics as the training data. That is, parameters of the concerned driver collected during previous driving sessions may be used as input training data. The (generative) AI may learn the patterns and structures of this previous data. For any following driving session, the new data may then be compared to the stored "expected" data. Additionally or alternatively, the input training data may include training data not related to a specific driver. The training data may represent parameters, patterns and characteristics generally expected for drivers that are not under the influence of impairing substances and are fit to drive. Such data can be captured by monitoring a plurality of drivers, for example, or may be based on generally accepted research findings, for example.

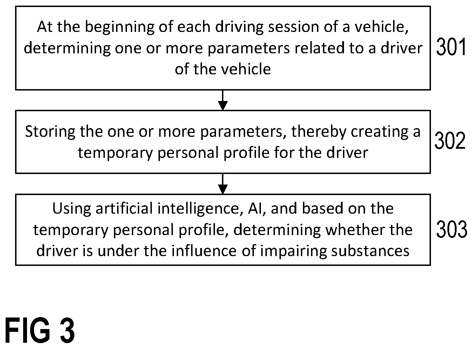

Now referring to Figure 3, a method according to embodiments of the disclosure is schematically illustrated by means of a flow diagram. The method comprises, at the beginning of each driving session of a vehicle 10, determining one or more parameters related to a driver of the vehicle 10 (step 301), storing the one or more parameters, thereby creating a temporary personal profile 52 for the driver (step 302), and using artificial intelligence, AI, and based on the temporary personal profile 52, determining whether the driver is under the influence of impairing substances (step 303).

The one or more parameters may include at least one physiological parameter of the driver, for example. The at least one physiological parameter may comprise at least one of a heart rate, a respiratory rate, an intensity of transpiration, and a body temperature of the driver. Additionally or alternatively, any other physiological parameters may be determined and taken into account when creating the temporary personal profile 52 of the driver. Additionally or alternatively, the one or more parameters may include at least one physical parameter of the driver. Physical parameters may include at least one of a size, a weight, an age, and a gender of the driver, for example.

According to some embodiments of the disclosure, the method may further comprise determining one or more vehicle parameters related to a current driving behavior, and storing the one or more vehicle parameters in the temporary personal profile 52 of the driver. According to some embodiments of the disclosure, the method may further comprise collecting one or more parameters related to the driver at least once during the driving session, and updating the temporary personal profile 52 of the driver.

Determining whether the driver is under the influence of impairing substances based on the temporary personal profile 52 may comprise evaluating the temporary personal profile 52 by means of the artificial intelligence, AI, thereby creating a current driver profile. Determining whether the driver is under the influence of impairing substances based on the temporary personal profile 52 may further comprise comparing the current driver profile to an expected driver profile (by means of the AI), wherein the expected driver profile comprises parameters related to a driver of the vehicle 10 and/or parameters of the vehicle 10 and collected during previous driving sessions, and/or the expected driver profile comprises parameters that are generally expected for drivers that are fit to drive.

Determining the one or more parameters related to the driver of the vehicle 10 may comprise receiving one or more parameters from one or more sensors 32, 34, 36, 38 arranged in the vehicle 10. According to some embodiments of the disclosure, determining the one or more parameters related to the driver of the vehicle 10 may further comprise receiving one or more parameters from one or more wearable sensors 60, from one or more fitness tracking devices 62, and/or from one or more permanent personal profiles of the respective passenger in a cloud or social network 64.

It may be understood, that the illustrated systems and methods are merely examples. While various embodiments of the invention have been described, it will be apparent to those of ordinary skill in the art that many more embodiments and implementations are possible within the scope of the invention. In particular, the skilled person will recognize the interchangeability of various features from different embodiments. Although these techniques and systems have been disclosed in the context of certain embodiments and examples, it will be understood that these techniques and systems may be extended beyond the specifically disclosed embodiments to other embodiments and/or uses and obvious modifications thereof. Accordingly, the invention is not to be restricted except in light of the attached claims and their equivalents.

The description of embodiments has been presented for purposes of illustration and description. Suitable modifications and variations to the embodiments may be performed in light of the above description or may be acquired from practicing the methods. The described arrangements are exemplary in nature, and may include additional elements and/or omit elements. As used in this application, an element recited in the singular and proceeded with the word "a" or "an" should be understood as not excluding plural of said elements, unless such exclusion is stated. Furthermore, references to "one embodiment" or "one example" of the present disclosure are not intended to be interpreted as excluding the existence of additional embodiments that also incorporate the recited features. The terms "first," "second," and "third," etc. are used merely as labels, and are not intended to impose numerical requirements or a particular positional order on their objects. The subject matter of the present disclosure includes all novel and non-obvious combinations and sub-combinations of the various systems and configurations, and other features, functions, and/or properties disclosed. The following claims particularly point out subject matter from the above disclosure that is regarded as novel and non-obvious.

## Claims

1. A method comprises,
at the beginning of each driving session of a vehicle (10), determining one or more parameters related to a driver of the vehicle (10),
storing the one or more parameters, thereby creating a temporary personal profile (52) for the driver, and
using artificial intelligence, AI, and based on the temporary personal profile (52), determining whether the driver is under the influence of impairing substances.

2. The method of claim 1, wherein the one or more parameters include at least one physiological parameter of the driver.

3. The method of claim 2, wherein the at least one physiological parameter comprises at least one of a heart rate, a respiratory rate, an intensity of transpiration, and a body temperature of the driver.

4. The method of any of claims 1 to 3, wherein the one or more parameters include at least one physical parameter of the driver.

5. The method of claim 4, wherein the one or more physical parameters include at least one of a size, a weight, an age, and a gender of the driver.

6. The method of any of the preceding claims, further comprising determining one or more vehicle parameters related to a current driving behavior, and storing the one or more vehicle parameters in the temporary personal profile (52) of the driver.

7. The method of any of the preceding claims, further comprising collecting one or more parameters related to the driver at least once during the driving session, and updating the temporary personal profile (52) of the driver.

8. The method of any of the preceding claims, wherein determining whether the driver is under the influence of impairing substances based on the temporary personal profile (52) comprises evaluating the temporary personal profile (52) by means of the artificial intelligence, AI, thereby creating a current driver profile.

9. The method of claim 8, wherein determining whether the driver is under the influence of impairing substances based on the temporary personal profile (52) further comprises comparing the current driver profile to an expected driver profile, wherein the expected driver profile comprises parameters related to a driver of the vehicle (10) and/or parameters of the vehicle (10) and collected during previous driving sessions, and/or the expected driver profile comprises parameters that are generally expected for drivers that are fit to drive.

10. The method of any of the preceding claims, wherein determining the one or more parameters related to the driver of the vehicle (10) comprises receiving one or more parameters from one or more sensors (32, 34, 36, 38) arranged in the vehicle (10).

11. The method of claim 10, wherein determining the one or more parameters related to the driver of the vehicle (10) further comprises receiving one or more parameters from one or more wearable sensors (60), from one or more fitness tracking devices (62), and/or from one or more permanent personal profiles of the respective passenger in a cloud or social network (64).

12. A driver assistance system (20) comprises a processor (22), the processor (22) configured to
at the beginning of each driving session of a vehicle (10), determine one or more parameters related to a driver of the vehicle (10),
store the one or more parameters, thereby creating a temporary personal profile (52) for the driver, and
using artificial intelligence, AI, and based on the temporary personal profile (52), determine whether the driver is under the influence of impairing substances.

13. The driver assistance system (20) of claim 12, wherein the driver assistance system (20) comprises or is coupled to one or more sensors (32, 34, 36, 38) arranged in the vehicle (10), and wherein determining the one or more parameters related to the driver of the vehicle (10) comprises receiving one or more parameters from the one or more sensors (32, 34, 36, 38).

14. The driver assistance system (20) of claim 13, wherein the one or more sensors (32, 34, 36, 38) comprise at least one of a camera (32), a radar sensor (34), a thermal imaging camera (36), and a weight sensor (38).

15. The driver assistance system of any of claims 12 to 14, further comprising or being coupled to a storage device (40), wherein the temporary personal profile (52) of the driver of the vehicle (10) is stored in the storage device (40).
